# EUROPEAN PATENT SPECIFICATION

(11) **EP 0 993 303 B1**
(45) Date of publication and mention of the grant of the patent: **21.05.2003**
(21) Application number: 98914197.3
(22) Date of filing: 26.03.1998
(51) Int. Cl.: A61K 38/11, A61K 7/00

(54) **SUBSTANCES IN ORDER TO ACHIEVE PREFERENCE AND CREATE ACCEPTANCE**
WIRKSTOFFE ZUR ERZEUGUNG VON VORZUG UND ANNAHME
SUBSTANCES DESTINEES A CREER PREFERENCE ET ACCEPTANCE

(30) Priority: 01.04.1997 SE 9701184
(43) Date of publication of application: 19.04.2000
(73) Proprietor: Uvnäs-Moberg, Kerstin, 182 62 Djursholm (SE); Lundeberg, Thomas, 181 31 Lidingö (SE)
(72) Inventor: Uvnäs-Moberg, Kerstin, 182 62 Djursholm (SE); Lundeberg, Thomas, 181 31 Lidingö (SE)
(74) Representative: Berg, Sven Anders
(86) International application number: SE9800560
(87) International publication number: WO98043662

(56) References cited:
- WO-A-98/43660
- WO-A-98/43661
- BEHAVIORAL AND NEURAL BIOLOGY, Volume 59, 1993, PIOTR POPIK et al., "Social Transmission of Flavored Tea Preferences: Facilitation by a Vasopressin Analog and Oxytocin", pages 63-68.

### SEQUENCE LISTING

<110> ENTRETECH MEDICAL AB

<120> SUBSTANCES IN ORDER TO ACHIEVE PREFERENCE AND CREATE ACCEPTANCE

<130> 51016

<140>
<141>

<160> 8

<170> PatentIn Ver. 2.1

<210> 1
<211> 9
<212> PRT
<213> Artificial Sequence

<220>
<221> MOD_RES
<222> (9)
<223> AMIDATION

<220>
<221> DISULFID
<222> (1)..(6)

<220>
<223> Xaa(3)=Cha

<220>
<223> Xaa(4)=Cit

<220>
<223> Description of Artificial Sequence:PEPTIDE

<400> 1
   Cys Tyr Xaa Xaa Asn Cys Pro Arg Gly
   1 5

<210> 2
<211> 9
<212> PRT
<213> Artificial Sequence

<220>
<221> MOD_RES
<222> (9)
<223> AMIDATION

<220>
<221> DISULFID
<222> (1)..(6)

<220>
<223> Description of Artificial Sequence:PEPTIDE

<400> 2
   Cys Tyr Val Thr Asn Cys Pro Leu Gly
   1 5

<210> 3
<211> 9
<212> PRT
<213> Artificial Sequence

<220>
<221> MOD_RES
<222> (9)
<223> AMIDATION

<220>
<221> DISULFID
<222> (1)..(6)

<220>
<223> Xaa(3)=Hoph

<220>
<223> Description of Artificial Sequence:PEPTIDE

<400> 3
   Cys Tyr Xaa Thr Asn Cys Pro Val Gly
   1 5

<210> 4
<211> 9
<212> PRT
<213> Artificial Sequence

<220>
<221> MOD_RES
<222> (9)
<223> AMIDATION

<220>
<221> DISULFID
<222> (1)..(6)

<220>
<223> Xaa(4)=Cit

<220>
<223> Description of Artificial Sequence:PEPTIDE

<400> 4
   Cys Tyr Phe Xaa Asn Cys Pro Leu Gly
   1 5

<210> 5
<211> 9
<212> PRT
<213> Artificial Sequence

<220>
<221> MOD_RES
<222> (9)
<223> AMIDATION

<220>
<221> DISULFID
<222> (1)..(6)

<220>
<223> Xaa(3)=Cha

<220>
<223> Xaa(8)=Hos

<220>
<223> Description of Artificial Sequence:PEPTIDE

<400> 5
   Cys Tyr Xaa Arg Asn Cys Pro Xaa Ala
   1 5

<210> 6
<211> 9
<212> PRT
<213> Artificial Sequence

<220>
<221> MOD_RES
<222> (9)
<223> AMIDATION

<220>
<221> DISULFID
<222> (1)..(6)

<220>
<223> Xaa(4)=Daba

<220>
<223> Xaa(8)=Daba

<220>
<223> Description of Artificial Sequence:PEPTIDE

<400> 6
   Cys Tyr Val Xaa Asn Cys Pro Xaa Ala
   1 5

<210> 7
<211> 9
<212> PRT
<213> Artificial Sequence

<220>
<221> MOD_RES
<222> (9)
<223> AMIDATION

<220>
<221> DISULFID
<222> (1)..(6)

<220>
<223> Xaa(3)=Hoph

<220>
<223> Xaa(4)=Daba

<220>
<223> Xaa(8)=Cit

<220>
<223> Description of Artificial Sequence:PEPTIDE

<400> 7
   Cys Tyr Xaa Xaa Asn Cys Pro Xaa Ala
   1 5

<210> 8
<211> 9
<212> PRT
<213> Artificial Sequence

<220>
<221> MOD_RES
<222> (9)
<223> AMIDATION

<220>
<221> DISULFID
<222> (1)..(6)

<220>
<223> Description of Artificial Sequence:PEPTIDE

<400> 8
   Cys Tyr Phe Arg Asn Cys Pro Val Ala
   1 5

## Description

The present invention relates to the use of substances with oxytocin activity in order to achieve preference and create acceptance. It also relates to a composition in order to create acceptance and/or preference comprising an effective concentration of at least one substance with oxytocin activity, together with at least one fragrance agent or flavouring and, optionally, at least one adjuvant.

### Background of the invention

Peptide hormones are substances naturally occurring in the body consisting of several amino acids. Such a peptide hormone is oxytocin. As a hormon this has a contraction developing effect on the smooth muscle in the uterus in mammals, wherein delivery is facilitated, as well as the muscles in milk glands, wherein milk ejection and milk production are facilitated. As a central stimulating substance oxytocin plays an important role in the interaction between mother and progeny in mammals. Accordingly, the oxytocin production in the mother results in acceptance and preference of her kid. This hormone is also important in order to a mammal kid being recognized by its mother, which results in that the kid searches and follows her. It has now been shown that oxytocin could be used, optionally, together with a flavouring or fragrance agent in different compositions in order to achieve preference and create acceptance. In comparison between perfumes with and without addition of oxytocin, respectively, in a number of subjects of an experiment, most of them prefer perfume with oxytocin added.

WO 95/01368 describes a procedure for preparation of cyclic peptides with disulfide groups, which procedure simplifies the synthesis and lowers the production costs. This procedure is useful in the preparation of oxytocin and vasopressin, and the corresponding derivatives and analogues thereof.

EP-A-689 452 describes a solid pharmaceutical composition for oral administration of small and medium-sized peptides, especially oxytocin, vasopressin and analogues thereof, and comprises, besides the peptide, a protease inhibitor, an enteric coating, and a pharmaceutically acceptable carrier.

WO 92/11287 describes new vasopressin and vasotocin derivatives. These derivatives have a strong affinity to oxytocin and V₁-vasopressin derivatives and are useful for the preparation of medicaments.

However, none of these known publications describes the use of oxytocin in order to achieve preference and create acceptance.

### Summary of the invention

The present invention relates to the use of a substance with oxytocin activity in order to achieve preference and create acceptance. It also relates to a composition in order to create acceptance and/or preference comprising an effective concentration of at least one substance with oxytocin activity, together with at least one fragrance agent or flavouring and, optionally, at least one adjuvant. Such a composition could be used in order to create acceptance in an animal of a progeny. It could also be used in order to achieve preference of hygienic and/or cosmetic products, and food, beverages, and stimulants etc.

### Detailed description of the invention

An object of the present invention is the use of a substance with an oxytocin like activity in order to achieve preference between a product with and without a substance, respectively, with the oxytocin like activity and to create acceptance. Examples of substances with oxytocin activity are the following compounds: i.e. W is Ile, X is Gln, Y is Leu, and Z is Gly in Claim 2 and 6 i.e. W is Ile, X is Gln, Y is Ile, and Z is Gly in Claim 2 and 6 i.e. W is Ile, X is Ser, Y is Ile, and Z is Gly in Claim 2 and 6 i.e. W is Ile, X is Gln, Y is Arg, and Z is Gly in Claim 2 and 6 i.e. W is Phe, X is Gln, Y is Arg, and Z is Gly in Claim 2 and 6.

Other substances with oxytocin activity could also be used, such as naturally occurring or artificially modified variants, analogues, and derivatives of oxytocin, mesotocin, isotocin, vasotocin, and vasopressin. Such substances could be obtained by addition, insertion, elimination, or substitution of at least one amino acid in these hormones. By substance with an oxytocin like activity is also understood precursors, metabolic derivatives, agonists, or analogues of the substances mentioned herein displaying the same properties. It could be ascertained that these variants are analogues of oxytocin, mesotocin, isotocin, or vasotocin by immunological methods, e.g. RIA (radio-immunoassay), IRMA (radiometric methods), RIST (radioimmunosorbent test), RAST (radioallergosorbent test). Oxytocin and vasopressin can be produced e.g. according to WO 95/01368 and oxytocin according to USP 2,938,891 and 3,076,797. There is also a possibility to create new compounds with oxytocin activity by means of computer simulation. Methods for computer simulation are known by a person skilled in the art, e.g. as described in EP 0660 210 A2. Eight new compounds have been created by means of computer simulation, namely the following peptides: i.e. W is Cha, X is Cit, Y is Arg, and Z is Gly in Claim 2 and 6 i.e. W is Val, X is Thr, Y is Leu, and Z is Gly in Claim 2 and 6 i.e. W is Hoph, X is Thr, Y is Val, and Z is Gly in Claim 2 and 6 i.e. W is Phe, X is Cit, Y is Leu, and Z is Gly in Claim 2 and 6 i.e. W is Cha, X is Arg, Y is Hos, and Z is Ala in Claim 2 and 6 i.e. W is Val, X is baba, Y is Daba, and Z is Ala in Claim 2 and 6 i.e. W is Hoph, X is Daba, Y is Cit, and Z is Ala in Claim 2 and 6 i.e. W is Phe, X is Arg, Y is Val, and Z is Ala in Claim 2 and 6,
wherein Cha stands for cyclohexylalanine, 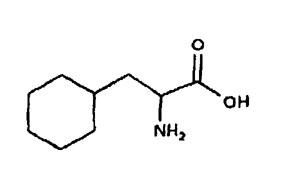 Hoph stands for homophenylalanine, 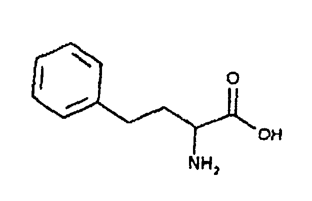 Cit stands for citruline, 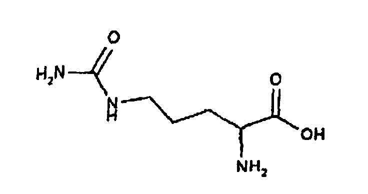 Daba stands for diaminobutyric acid, and 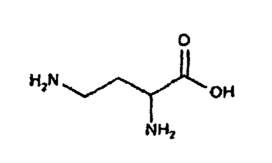 Hos stands for homoserine.

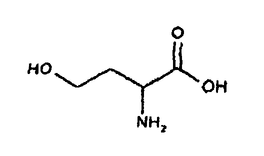

The invention also relates to the peptides mentioned above in both D- and L-form. Especially the invention relates to the L-form. By inversion of the peptide sequence thereof, the D-form could be converted to the L-form. The effect of the D- and L-forms are the same. These and the peptides above can be produced by methods known to a person skilled in the art, e.g. according to Merrifield, P.B., "Solid Phase Synthesis", *Angew. Chemie,* **1985**, No. 97, p. 801.

It could be ascertained that those compounds prepared by computer simulation have oxytocin effect by tests, such as described in the appended Examples. Preferably, oxytocin is used.

Another object of the invention is a composition in order to create acceptance and/or preference comprising an effective concentration of at least one substance with oxytocin activity, together with at least one fragrance agent or flavouring and, optionally, at least one adjuvant. An effective concentration could be determined by a person skilled in the art. The concentration of the substance with oxytocin activity could be 0.5-500 IU, especially 1-100 IU. Examples of substances with oxytocin activity are oxytocin derivatives, such as oxytocin, mesotocin, isotocin, SEQ ID NO: 2, SEQ ID NO: 3, SEQ ID NO: 4, and SEQ ID NO: 8; vasopressin derivatives, such as vasotocin, vasopressin, SEQ ID NO: 1, SEQ ID NO: 5, SEQ ID NO: 6, and SEQ ID NO: 7. By adjuvants are meant conventional adjuvants that strengthen the effect, or carrier substances. It is preferred that the fragrance agent and/or the adjuvant are selected from the group consisting of hygienic and/or cosmetic products, such as eau-de-Cologne, eau-de-toilette, perfume, make-up, soap, shower cream, hair conditioning agents, such as shampoos, conditioning agents, creams, lotions, and ointments; stimulants, such as cigars, cigarettes, tobacco, and chewing-tobacco. For example, such a composition could be used in order to create acceptance. An example thereof is the habituation of a cow on a milking machine. When a cow, which has recently calved, is allowed to suckle her calf, the imprinting thereof will be so strong that the cow could have some trouble in accepting a milking machine. By application of the composition hereinabove of the milking machine, the acceptance thereof by the cow could be facilitated. Another example is childrens' weaning from their mother's breast when breast-feeding and conversion to a feeding bottle, and the contrary. By application of the composition hereinabove on a feeding bottle/the mother's breast, the acceptance thereof by the child could be facilitated.

The effect of oxytocin could be tested by measuring the contraction effect thereof in the uterus or the bindning of oxytocin to receptors in the uterus. These receptors are not necessarily the same as in the case to achieve preference and create acceptance. Therefore, it is suitable to measure the effect of oxytocin with a trial panel in order to ascertain these effects.

The invention is illuminated by the following Examples. These Examples are only illustrative and are not intended to limit the invention in any way whatsoever.

By the expression "comprising" we understand including but not limited to. Thus, other non-mentioned substances, additives or carriers may be present.

### Examples

In a trial to ascertain the effect of oxytocin a trial panel compared the odour of a paper sheet sprayed five times with either of the following mixtures: mixture A (Perfume Chanel, no 5) and mixture B (Perfume Chanel, no 5 + Syntocinon® in the form of a nasal spray with 4 IU oxytocin/ml nasal spray).

### Example 1

The trial panel consisted of 40 male students, aged 19-32 years. Every one was a non-smoker and not afflicted with a cold. Of these, 29 preferred mixture B, 8 preferred mixture A, and 3 did not notice any odour difference between mixture A and mixture B.

### Example 2

The trial panel consisted of 38 female students, aged 19-32 years. Every one was a non-smoker and not afflicted with a cold. Of these, 27 preferred mixture B, 10 preferred mixture A, and 1 did not notice any odour difference between mixture A and mixture B.

From the Examples it could be seen that addition of oxytocin creates preference in a choice between perfumes with and without oxytocin, respectively.

## Claims

1. Use of a substance with oxytocin activity in order to achieve preference between a product with and without a substance with oxytocin activity, respectively, and/or in order to create acceptance.

2. Use according to Claim 1, **characterized in that** the substance is selected from the group consisting of the following oxytocin derivatives: wherein
W is selected from the group consisting of Ile, Val, Hoph, and Phe,
X is selected from the group consisting of Gln, Ser, Thr, Cit, and Arg,
Y is selected from the group consisting of Leu, Ile, and Val,
Z is selected from the group consisting of Gly and Ala;
and the following vasopressin derivatives: wherein
W is selected from the group consisting of Ile, Phe, Cha, Val, and Hoph,
X is selected from the group consisting of Gln, Cit, Arg, and Daba,
Y is selected from the group consisting of Arg, Hos, Daba, and Cit,
Z is selected from the group consisting of Gly and Ala.

3. Use according to Claim 1-2, **characterized in that** the substance is selected from the group consisting of the following oxytocin derivatives:
oxytocin,
mesotocin,
isotocin,
and and the following vasopressin derivatives:
vasotocin,
vasopressin,

4. Composition in order to create acceptance and/or preference, **characterized in that** it comprises an effective concentration of at least one substance with oxytocin activity, together with at least one fragrance agent or flavouring and, optionally, at least one adjuvant.

5. Composition according to Claim 4, **characterized in that** the effective concentration is 0.5-500 IU, especially 1-100 IU.

6. Composition according to Claim 4, **characterized in that** the substance is selected from the group consisting of the following oxytocin derivatives: wherein
W is selected from the group consisting of Ile, Val, Hoph, and Phe,
X is selected from the group consisting of Gln, Ser, Thr, Cit, and Arg,
Y is selected from the group consisting of Leu, Ile, and Val,
Z is selected from the group consisting of Gly and Ala;
and the following vasopressin derivatives: wherein
W is selected from the group consisting of Ile, Phe, Cha, Val, and Hoph,
X is selected from the group consisting of Gln, Cit, Arg, and Daba,
Y is selected from the group consisting of Arg, Hos, Daba, and Cit,
Z is selected from the group consisting of Gly and Ala.

7. Composition according to Claim 6, **characterized in that** the substance is selected from the group consisting of the following oxytocin derivatives:
oxytocin,
mesotocin,
isotocin,
and and the following vasopressin derivatives:
vasotocin,
vasopressin,

8. Composition according to Claim 4, **characterized in that** the fragrance agent and/or the adjuvant are selected from the group consisting of hygienic and/or cosmetic products, such as eau-de-Cologne, eau-de-toilette, perfume, make-up, soap, shower cream, hair conditioning agents, such as shampoos, conditioning agents, creams, lotions, and ointments; stimulants, such as cigars, cigarettes, tobacco, and chewing-tobacco.

## Patentansprüche

1. Verwendung einer Substanz mit Oxytocin-Aktivität zum Erreichen von Präferenz zwischen einem Produkt mit einer bzw. ohne eine Substanz mit Oxytocin-Aktivität und zum Schaffen von Akzeptanz.

2. Verwendung gemäß Anspruch 1, **dadurch gekennzeichnet, daß** die Substanz aus der Gruppe ausgewählt ist, die aus den folgenden Oxytocin-Derivaten: worin
W aus der Gruppe ausgewählt ist, die aus Ile, Val, Hoph und Phe besteht,
X aus der Gruppe ausgewählt ist, die aus Gln, Ser, Thr, Cit und Arg besteht,
Y aus der Gruppe ausgewählt ist, die aus Leu, Ile und Val besteht,
Z aus der Gruppe ausgewählt ist, die aus Gly und Ala besteht;
und den folgenden Vasopressin-Derivaten besteht: worin
W aus der Gruppe ausgewählt ist, die aus Ile, Phe, Cha, Val und Hoph besteht,
X aus der Gruppe ausgewählt ist, die aus Gln, Cit, Arg und Daba besteht,
Y aus der Gruppe ausgewählt ist, die aus Arg, Hos, Daba und Cit besteht,
Z aus der Gruppe ausgewählt ist, die aus Gly und Ala besteht.

3. Verwendung gemäß Anspruch 1 bis 2, **dadurch gekennzeichnet, daß** die Substanz aus der Gruppe ausgewählt ist, die aus den folgenden Oxytocin-Derivaten:
Oxytocin,
Mesotocin,
Isotocin,
und und den folgenden Vasopressin-Derivaten besteht:
Vasotocin,
Vasopressin,
und

4. Zusammensetzung zur Schaffung von Akzeptanz und/oder Präferenz, **dadurch gekennzeichnet, daß** sie eine wirksame Konzentration wenigstens einer Substanz mit Oxytocin-Aktivität zusammen mit wenigstens einem Duftstoff oder Aromastoff und gegebenenfalls wenigstens einem Adjuvans umfaßt.

5. Zusammensetzung gemäß Anspruch 4, **dadurch gekennzeichnet, daß** die wirksame Konzentration 0,5 bis 500 IE ist, speziell 1 bis 100 IE.

6. Zusammensetzung gemäß Anspruch 4, **dadurch gekennzeichnet, daß** die Substanz aus der Gruppe ausgewählt ist, die aus den folgenden Oxytocin-Derivaten: worin
W aus der Gruppe ausgewählt ist, die aus Ile, Val, Hoph und Phe besteht,
X aus der Gruppe ausgewählt ist, die aus Gln, Ser, Thr, Cit und Arg besteht,
Y aus der Gruppe ausgewählt ist, die aus Leu, Ile und Val besteht,
Z aus der Gruppe ausgewählt ist, die aus Gly und Ala besteht;
und den folgenden Vasopressin-Derivaten besteht: worin
W aus der Gruppe ausgewählt ist, die aus Ile, Phe, Cha, Val und Hoph besteht,
X aus der Gruppe ausgewählt ist, die aus Gln, Cit, Arg und Daba besteht,
Y aus der Gruppe ausgewählt ist, die aus Arg, Hos, Daba und Cit besteht,
Z aus der Gruppe ausgewählt ist, die aus Gly und Ala besteht.

7. Zusammensetzung gemäß Anspruch 6, **dadurch gekennzeichnet, daß** die Substanz aus der Gruppe ausgewählt ist, die aus den folgenden Oxytocin-Derivaten:
Oxytocin,
Mesotocin,
Isotocin,
und und den folgenden Vasopressin-Derivaten besteht:
Vasotocin,
Vasopressin,
und

8. Zusammensetzung gemäß Anspruch 4, **dadurch gekennzeichnet, daß** der Duftstoff und/oder das Adjuvans aus der Gruppe ausgewählt sind, die aus Hygieneund/oder Kosmetikprodukten, wie Eau de Cologne, Eau de Toilette, Parfüm, Make-up, Seife, Duschcreme, Haarspülungen wie Shampoos, Spülungen, Cremes, Lotionen und Salben; Genußmitteln, wie Zigarren, Zigaretten, Tabak und Kautabak, besteht.

## Revendications

1. Utilisation d'une substance ayant une activité ocytocine afin d'obtenir une préférence entre un produit avec et un produit sans substance ayant une activité ocytocine, respectivement, et/ou afin de créer une acceptation.

2. Utilisation selon la revendication 1, **caractérisée en ce que** la substance est choisie dans le groupe constitué des dérivés de l'ocytocine suivants : dans lesquels
W est choisi dans le groupe constitué de Ile, Val, Hoph et Phe,
X est choisi dans le groupe constitué de Gln, Ser, Thr, Cit et Arg,
Y est choisi dans le groupe constitué de Leu, Ile et Val,
Z est choisi dans le groupe constitué de Gly et Ala ;
et des dérivés de la vasopressine suivants : dans lesquels
W est choisi dans le groupe constitué de Ile, Phe, Cha, Val et Hoph,
X est choisi dans le groupe constitué de Gln, Cit, Arg et Daba,
Y est choisi dans le groupe constitué de Arg, Hos, Daba et Cit,
Z est choisi dans le groupe constitué de Gly et Ala.

3. Utilisation selon la revendication 1 ou 2, **caractérisée en ce que** la substance est choisie dans le groupe constitué des dérivés de l'ocytocine suivants :
l'ocytocine,
la mésotocine,
l'isotocine,
et et des dérivés de la vasopressine suivants :
vasotocine,
vasopressine

4. Composition afin de créer une acceptation et/ou une préférence, **caractérisée en ce qu'**elle comprend une concentration efficace d'au moins une substance ayant une activité pour l'ocytocine, avec au moins un parfum ou un agent aromatisant et, facultativement, au moins un adjuvant.

5. Composition destiné à la revendication 4, **caractérisée en ce que** la concentration efficace est comprise dans la plage allant de 0,5 à 500 UI, en particulier allant de 1 à 100 UI.

6. Composition selon la revendication 4, **caractérisée en ce que** la substance est choisie dans le groupe constitué des dérivés de l'ocytocine suivants dans lesquels
W est choisi dans le groupe constitué de Ile, Val, Hoph et Phe,
X est choisi dans le groupe constitué de Gln, Ser, Thr, Cit et Arg,
Y est choisi dans le groupe constitué de Leu, Ile et Val,
Z est choisi dans le groupe constitué de Gly et Ala ;
et des dérivés de la vasopressine suivant : dans lesquels
W est choisi dans le groupe constitué de Ile, Phe, Cha, Val et Hoph,
X est choisi dans le groupe constitué de Gln, Cit, Arg et Daba,
Y est choisi dans le groupe constitué de Arg, Hos, Daba et Cit,
Z est choisi dans le groupe constitué de Gly et Ala.

7. Composition selon la revendication 6, **caractérisée en ce que** la substance est choisie dans le groupe constitué des dérivés de l'ocytocine suivants :
l'ocytocine,
la mésotocine,
l'isotocine,
et et des dérivés de la vasopressine suivants :
vasotocine,
vasopressine

8. Composition selon la revendication 4, **caractérisée en ce que** le parfum et/ou l'adjuvant sont choisis dans le groupe constitué de produits cosmétiques et/ou hygiéniques tels que l'eau de Cologne, l'eau de toilette, le parfum, le maquillage, le savon, le gel douche crème, les agents de revitalisation capillaire tels que les shampooings, les agents après-shanpooing, les crèmes, les lotions et les pommades ; de stimulants tels que les cigares, les cigarettes, le tabac et les gommes à mâcher à la nicotine.
